# EUROPEAN PATENT SPECIFICATION

(11) **EP 3 723 630 B1**
(45) Date of publication and mention of the grant of the patent: **04.11.2026**
(21) Application number: 18826232.3
(22) Date of filing: 12.12.2018
(51) Int. Cl.: A61B 17/17, A61B 17/88

(54) **GUIDE FOR POSITIONING A RESURFACING HEAD IMPLANT**
FÜHRUNG ZUR POSITIONIERUNG EINES OBERFLÄCHENERSATZIMPLANTATS
GUIDE POUR POSITIONNER UN IMPLANT DE RESURFAÇAGE DE TÊTE

(30) Priority: 13.12.2017 GB 201720804
(43) Date of publication of application: 21.10.2020
(73) Proprietor: Embody Orthopaedic Limited, London W12 0BZ (GB)
(72) Inventor: WOZENCROFT, Robert, Micheal, London N1 1JT (GB)
(74) Representative: Allen, Caroline Margaret
(86) International application number: PCT/EP2018/084633
(87) International publication number: WO 2019/115648

(56) References cited:
- EP-A1- 1 477 120
- EP-A1- 1 588 668
- WO-A1-2010/148103
- US-A1- 2010 114 101

## Description

### Background

During a hip resurfacing operation, the head of a patient's femur is normally retained and capped with a head implant with a spherical bearing of a similar size to the natural joint. The bone socket in the pelvis is reamed to approximately a hemispherical shape and a thin walled cup implant is fitted to completely reline the hip joint with an artificial bearing. In order to regain natural range of motion and to ensure the bearing functions well over a long period of time, it is very important to position both the head and cup implants correctly relative to the natural bone and soft tissue structures. This is particularly challenging in a diseased hip where the head of the femur is often misshapen and the acetabular socket may have migrated slightly out of position. This can be misleading when it comes to positioning the implant components. Furthermore, the surgeon has limited access and visibility during the operation, so it can be difficult to visualise anatomical landmarks and to judge the orientation of the bones.

When the surgeon positions a resurfacing head implant on the femur bone the following factors are generally considered:
1. Femoral head/ neck size: The choice of head implant size should be approximately the same as the natural head size, whilst at the same time there must be enough bone available on the head to support the implant fully and it is vital that the cutters used to prepare the head do not encroach into the femoral neck (known as notching) as it weakens the bone and can cause femoral neck fracture postoperatively.
2. Varus/ valgus angle: This is the angle between the femur shaft axis and the head implant axis and should be in the range 135 - 145 degrees so that the load is transferred through the implant without putting undue stress on the bone.
3. Version angle: This is the forward tilt angle of the femoral neck relative to the frontal anatomical plane. It is unique for each patient and usually within the range 15 - 25 degrees. The surgeon will try to place the head axis according to the natural femoral neck version angle for a specific patient.

Patient specific guides are sometimes used in joint replacement operations to help position implants. They are defined and constructed preoperatively based on three-dimensional digital images of the patient's joints. The digital images of the patient's joint can be reconstructed from medical scans of the patient using commercially available CAD (Computer Aided Design) and/or other specific planning software. The surgeon or skilled technician interacts with the software to place the implant in the desired positions relative to the patient's scanned bones. A patient matched guide is then defined and constructed using rapid prototyping techniques such as additive manufacture. These guides are devised to fit exactly with a patients exposed bone surfaces intraoperatively so that the implants can be directed exactly to their planned positions. An advantage of a patient specific head guide for the resurfacing head is that multifactorial variables listed (1-3) above are all resolved simultaneously according to a pre-operative plan as the guide fits exactly into a distinct position on the femur bone.
EP 1,477,120 A1 discloses a locating tool, the locating tool comprising first and second elongate handles respectively merging with first and second arcuate members, the first and second handle being designed with tapers to allow for the first and second arms to rotate freely about a hinge; the locating tool being moveable between an open position and a closed position, wherein in the open position the first and second arcuate members are sufficiently spaced apart to permit the receipt of a femoral neck and in the closed position the first and second arcuate members are configured to closely fit around the femoral neck.

### Statement of Invention

The present invention is a guide for positioning a resurfacing head implant into the appropriate position and orientation on the femur bone. In the context of the invention, the word "proximal" is used to refer to a part of the guide which is closer to the patient's femur shaft during use, and the word "distal" is used to refer to a part of the guide which is further away from the patient's femur shaft during use.

The present invention relates to a femoral head guide clamp comprising:
(a) jaws comprising a ring forming an aperture, the aperture shaped such that (i) the jaws have a closed position in which the aperture fits around a femoral neck of a patient but is too small to accept a femoral head of a patient, and (ii) the jaws are movable from the closed position to an open position in which the aperture is expanded such that it is large enough to allow the femoral head to pass through; wherein the ring comprises a discontinuity, the ring being openable at the discontinuity when the jaws are moved towards the open position in order to allow the femoral head to pass through the ring, and
(b) two opposing arms that extend from the jaws in a direction perpendicular to the plane of the ring and which have a proximal section in which the arms are substantially parallel in a side-by-side arrangement, and a distal section in which the arms extend away from each other such that moving the two distal sections towards each other moves the jaws towards the open position.
In particular, the jaws may include a lockable member for securing the jaws in the closed position.

More particularly, the jaws may extend away from the ring in a direction which is substantially coplanar with the ring, and then connect to the arms which extend distally in a direction which, in use, is towards a user of the femoral head guide clamp.

In particular, the distal section of at least one of the two opposing arms may comprise protrusions which are shaped to be received by corresponding depressions on the opposing arm when the two distal sections are moved towards each other to move the jaws towards the open position.

In particular, the femoral head guide clamp may additionally comprise a guide for a drill or cutter. More particularly, the guide for a drill or cutter may be removable. Even more particularly, the removable guide may be slidably attachable to the two opposing arms of the femoral head guide clamp. More particularly, the removable guide may be slidably attachable to the proximal sections of the arms. In particular, the two opposing arms may each comprise an elongate rail extending along each arm and which are shaped to mate with two corresponding elongate recesses on the removable guide. More particularly, the removable guide may comprise a lockable member for securing the removable guide to the femoral head guide clamp. Even more particularly, the removable guide may comprise a lockable member for securing the removable guide to the femoral head guide clamp, the lockable member being able to press the elongate rails against a wall of each of the elongate recesses in order to secure the removable guide to the femoral head guide clamp.

In particular, one or both of the femoral head guide clamp and the removable guide may be a patient specific instrument. More particularly, the jaws may be provided with one or more neck protrusions which are shaped such that the jaws are only moveable to the closed position when around a femoral neck of a specific patient and when the femoral head guide clamp is in a predefined orientation relative to that patient's femur. Even more particularly, the neck protrusions may be shaped as substantially flat ribs. More particularly, the ring may comprise a discontinuity and the neck protrusions may be provided on the ring. Even more particularly, the neck protrusions may extend in one or more of the following directions: above a plane of the ring, below a plane of the ring, towards a centre of the ring, and away from a centre of the ring.

More particularly, the guide for a drill or cutter may comprise one or more head protrusions which, in use, extend towards a femoral head of a specific patient and which are shaped to conform to the surface of the femoral head when the jaws are closed around a femoral neck of the patient. Even more particularly, the head protrusions may be shaped as substantially flat ribs which additionally extend axially away from the removable guide.

In particular, one or both of the femoral head guide clamp and the removable guide may be formed from a resilient material. More particularly, the resilient material may be a polyamide.

Further described herein is a method of clamping a femoral head guide clamp as described above to a femur, the method comprising the steps of:
(a) opening the jaws of the femoral head guide clamp,
(b) passing the femoral head through the aperture, and
(c) closing the jaws of the femoral head guide clamp.

In particular, the jaws may include a lockable member for securing the jaws in the closed position and the method may comprise the additional step, after step (c), of:
(d) locking the jaws in the closed position.

More particularly, the method may additionally include the step of attaching a guide for a drill or cutter to the femoral head guide clamp. Even more particularly, the two opposing arms may each comprise an elongate rail extending along each arm and which are shaped to mate with two corresponding elongate recesses on the removable guide, and the step of attaching comprising sliding the removable guide onto the elongate rails. In particular, the attaching step may be carried out after step (c) or after step (d) and may comprise contacting a femoral head with the removable guide.

Further described herein is a computer-readable medium having computer-executable instructions adapted to cause a 3D printer to print a femoral head guide clamp and/or a removable guide as described above.

### Detailed description

A purpose of the guide is to position a drilled hole in the femur bone which defines the orientation and position of a resurfacing head implant. Two embodiments of the guide are described herein, a generic guide which comes in a range of sizes and may be used for any patient, and a patient specific guide which is tailored to fit a specific patient's anatomy. Both embodiments are preferably manufactured in plastic (for example polyamide) using an additive manufacturing process. The skilled person is aware of several types of additive manufacturing processes, for example selective laser sintering (SLS) or stereolithography (SLA). Alternatively, these parts could be moulded in plastic, for example by injection moulding or cold curing casting resin. Alternatively, they could be manufactured in plastic by any other means.

The guide may be made up of two parts, a femoral head guide clamp and a removable drill guide. The head guide clamp includes a circular ring, which, in use is positioned around the neck of the femur bone. The ring includes opening jaws, where the ring joins to two opposing arms. These arms extend approximately perpendicular to the plane of the ring. The arms have a proximal section where they are parallel in a side-by-side arrangement and a distal section where the arms form handles which move away from one another.

The head guide clamp is manufactured from a resilient material (for example polyamide) and can, for example, be flexed open in one of two ways to enable it to be positioned around the neck of the femur bone. A first method is to prise the handles apart by holding one handle in each hand. An opening movement allows the ring to expand open and become large enough for it to pass over the femoral head. When the opening grip is relaxed, the ring closes around the femoral neck. A second method is to squeeze the distal handle section of the opposing arms together which, due to the shape of the arms including a fulcrum position approximately halfway along (at the intersection of the proximal section and distal section), causes the arms at the proximal end to gape apart and the ring to expand open large enough for it to pass over the femoral head. This second method is convenient for the surgeon because it can be done with one hand.

The drill guide may include a cylindrical boss with an aperture for guiding a drill, an elongated section for connecting it to the head guide clamp and a cam locking lever. When assembled the drill guide may be slidably connected to the proximal section of the head guide clamp. The cam locking lever can allow the drill guide to be locked in a variety of proximal distal positions on the head guide clamp. In use, the drill guide can be moved to a position where the proximal section of the cylindrical boss is as close as possible and preferably in contact with the femoral head. Furthermore, this locking mechanism provides a means for clamping the guide securely between the femoral head and femoral neck.

Preferably a drill tube with a spiked end is inserted into the aperture of the drill guide. The spiked end slides down to contact the femoral head and the spikes can embed into the bone slightly (preferable the drill tube is manufactured in metal such as stainless steel). Then a drill (for example 5 mm in diameter) may be passed through the drill tube and a hole is drilled into the femur bone corresponding to the central longitudinal axis of the resurfacing head implant. The spiked end can ensure that the drill enters the bone without deviating off line.

In the patient specific version of the guide, additional features may be included to make the head guide clamp and drill guide fit a specific patient's femur bone. On the head guide clamp the ring can include protrusions shaped to engage with the bone surface on the patient's femoral neck in several positions, thus providing an exact fit of the guide in a predetermined orientation relative to the patient's femur. Furthermore, on the drill guide, protrusions may be included at the proximal end of the cylindrical boss. These protrusions can be shaped to engage with the surfaces of the patient's femoral head in several positions, thus further enhancing the exact fit of the guide in a predetermined orientation relative to the patient's femur. These opposing features may also provide clamping means between the femoral head and neck.

In an alternative embodiment, the removable drill guide may incorporate a larger diameter cylindrical boss with a large aperture and removable inner collar. The inner collar can be used to guide a small diameter drill (for example a 5mm drill) into the femur bone but when the inner sleeve is removed, a larger counterbore cutter (for example a diameter 30 mm cutter) may be introduced and guided by the large aperture in the cylindrical boss. Furthermore, the distal end of the cylindrical boss can provide an end stop for the large counter bore cutter. Therefore, provided the patient specific protrusions are in contact with the femoral head surface, the depth of counterbore can be guided to a predetermined depth relative to the patient's femur bone. The counterbore surface coincides with the internal end face of the resurfacing head implant, so that the proximal distal position of the resurfacing head implant may be precisely controlled by the guide. In this case both the proximal distal axis and proximal distal position may be controlled by the guide.

Examples of the invention will now be described by referencing the accompanying drawings, which are not intended to limit the scope of the invention claimed, in which:
**Figure 1** shows the assembled head guide according to one embodiment of the invention,
**Figure 2** shows the head guide clamp part of the head guide of Figure 1 in isolation and in the closed position,
**Figure 3** shows the head guide clamp of Figure 2 in an opened position,
**Figure 4** shows the drill guide part of head guide of Figure 1 in isolation,
**Figure 5** is an exploded view of the head guide of Figure 1,
**Figure 6** is the head guide of Figure 1 assembled in position on a femur bone,
**Figure 7** is a cross sectioned view of the drill guide of Figure 4,
**Figure 8** is a view of the head guide clamp of Figure 2, cross sectioned through the ring, jaws and retaining feature,
**Figure 9** is a view of the head guide of Figure 1, cross sectioned at position A-A,
**Figure 10** shows an alternative patient specific version of the head guide,
**Figure 11** shows the head guide clamp part of the head guide of Figure 10 in isolation and in a slightly opened position,
**Figure 12** is the head guide of Figure 10 assembled in position on a femur bone,
**Figure 13** shows the head guide clamp of Figure 11, in an opened position, and
**Figure 14** shows an alternative embodiment of a patient specific drill guide.

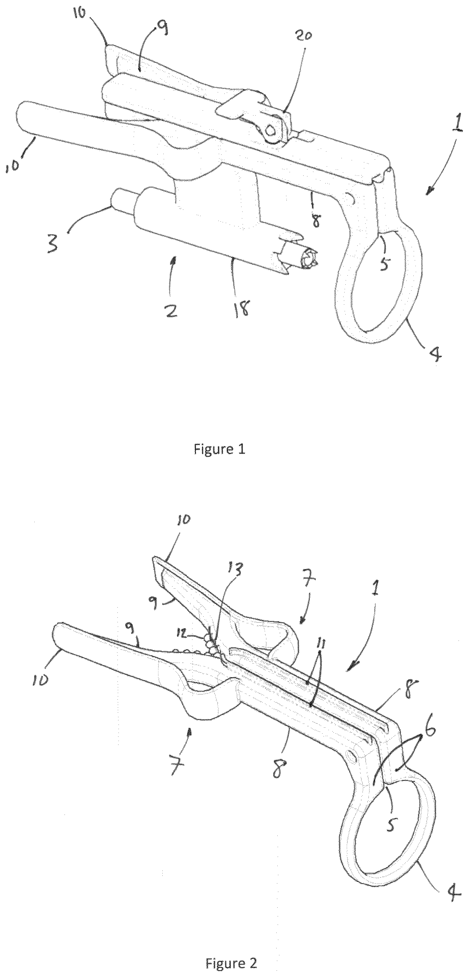

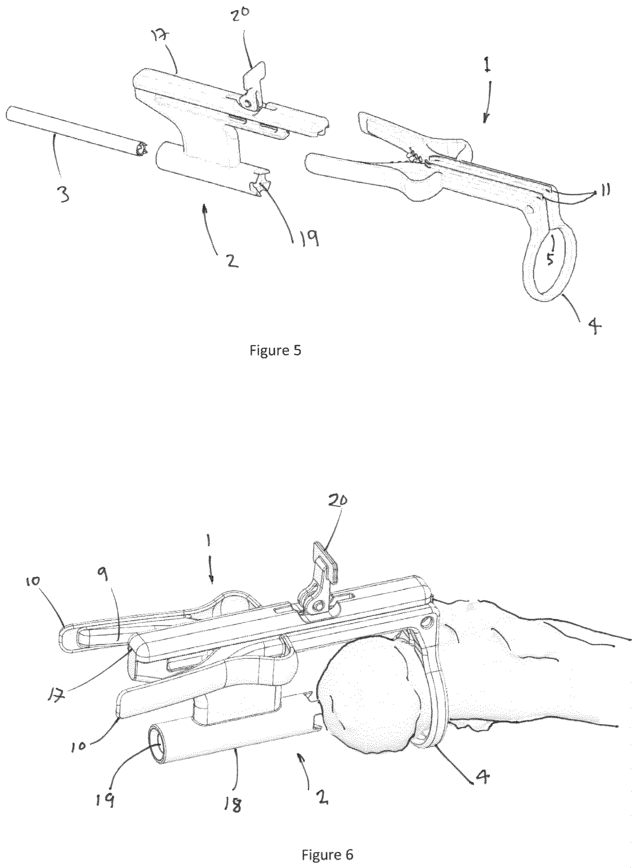

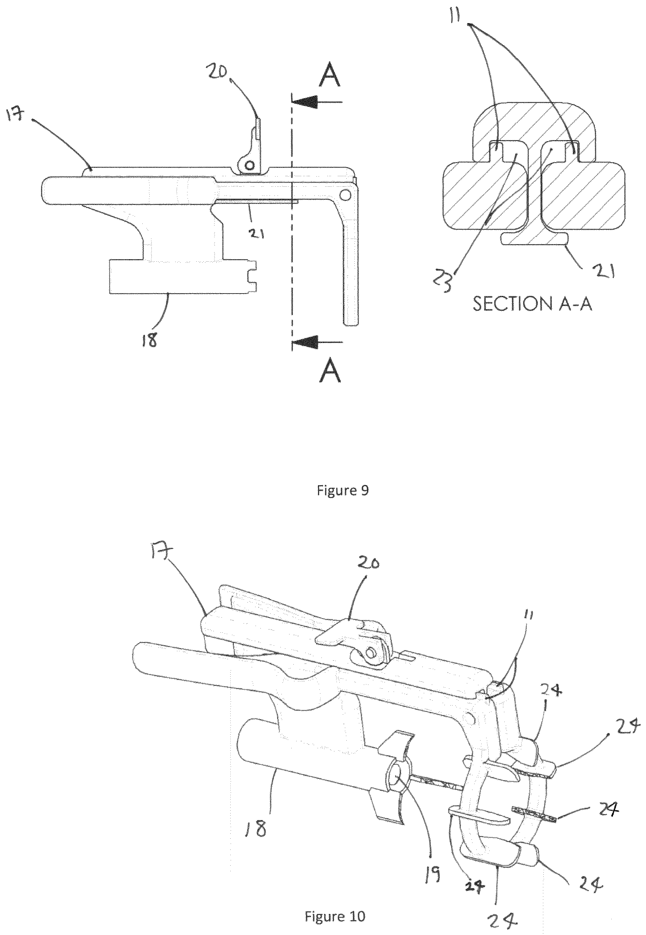

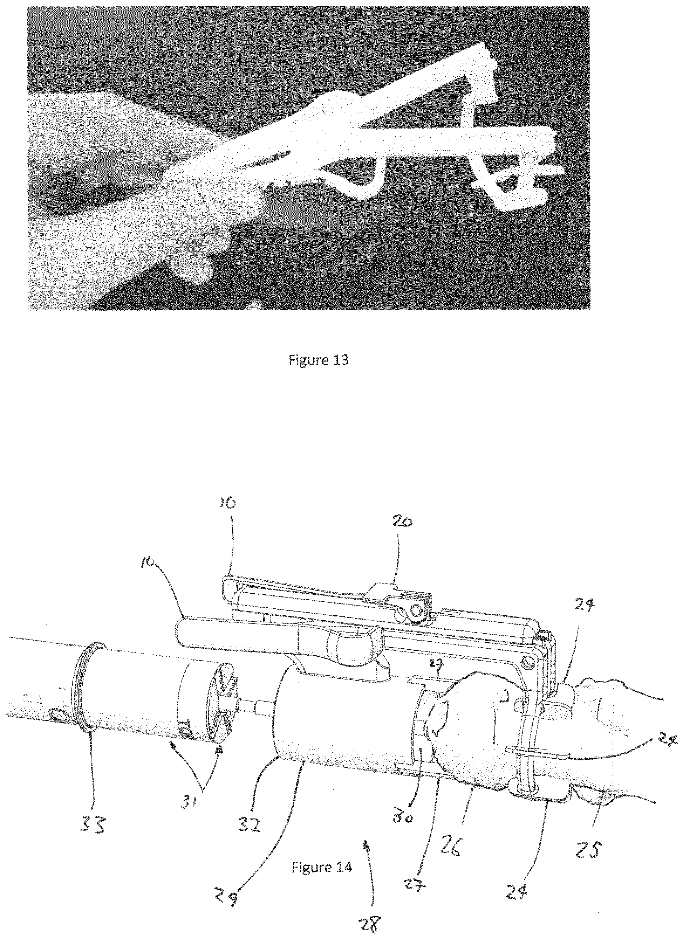

A generic version of the head guide is shown in Figures 1 - 9. This version comes in a range of sizes, one for each size of resurfacing head implant (for example 10 sizes). A patient specific version of the head guide is shown in Figures 10 - 13 which is manufactured to fit a specific patient's anatomy. An alternative embodiment of a patient specific drill guide is shown in Figure 14.

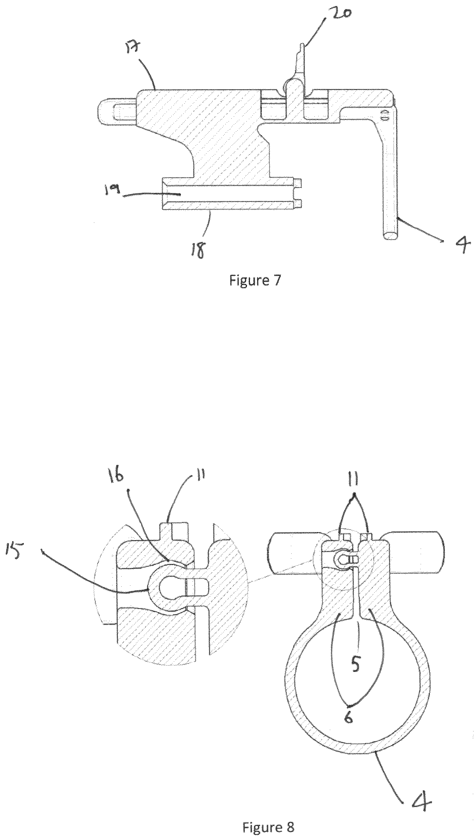

In Figures 1 - 5 it can be seen that the head guide comprises a head guide clamp [1], removable drill guide [2] and optionally a spiked tube [3]. The head guide clamp [1] comprises a circular ring [4] with discontinuity [5] and two jaws [6] extending away from the discontinuity [5]. Extending from the jaws are two opposing arms [7], each arm comprises a proximal section [8] where the arms are substantially parallel and distal section [9] where the arms extend away from one another forming handles [10]. The proximal sections of the arms [9] each incorporate an elongated rail [11]. The distal section of the arms incorporates protrusions [12] which are shaped to receive corresponding depressions [13] on the opposing arm when the arms are moved towards each other in a similar way to that shown in Figure 13 in relation to the patient specific version. In Figure 8, a retaining feature is shown comprising a spherical stud [15] on one arm and an aperture [16] on the opposing arm, this feature temporarily retains the opposing arms in the closed position.

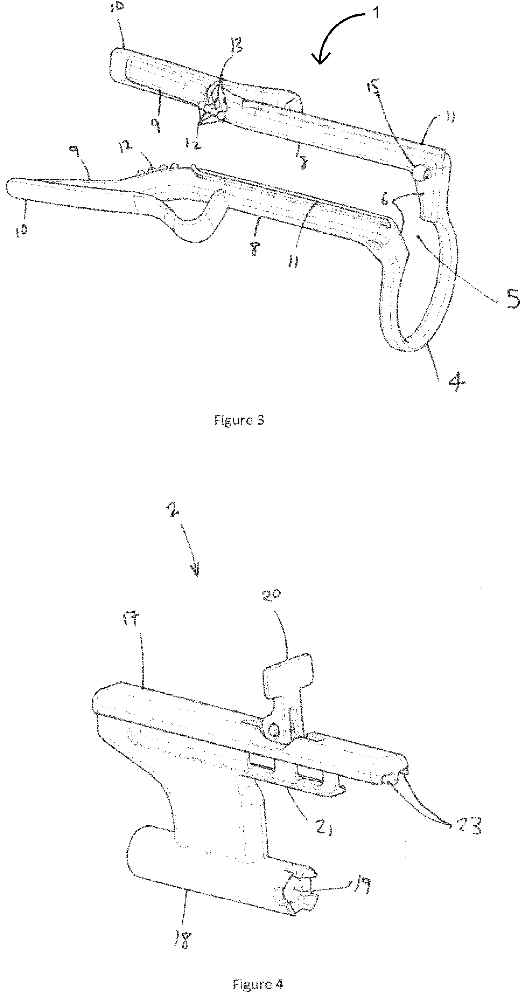

The removable drill guide [2] comprises body [17] and a cylindrical boss [18] with aperture [19]. The body incorporates a cam locking lever [20]. In Figures 4 and 7 the cam locking lever [20] is shown in the unlocked position. When the locking lever [20] is rotated through 90 degrees to the lock position (as shown in Figure 10 in relation to the patient specific version), inferior flange [21] of the body [17] is pulled upwards (ie towards locking lever [20]) to clamp the drill guide [2] in a certain proximal distal position when assembled on the head guide clamp [1]. In Figure 9 (Section A-A) the connection between head guide clamp and drill guide is shown, consisting of elongated rails [11] on the head guide clamp and elongated recesses [23] on the drill guide [2]. Sliding movement occurs between the head guide clamp [1] and drill guide [2] in a proximal distal direction and this movement is locked when the cam locking lever [20] is in the locked position.

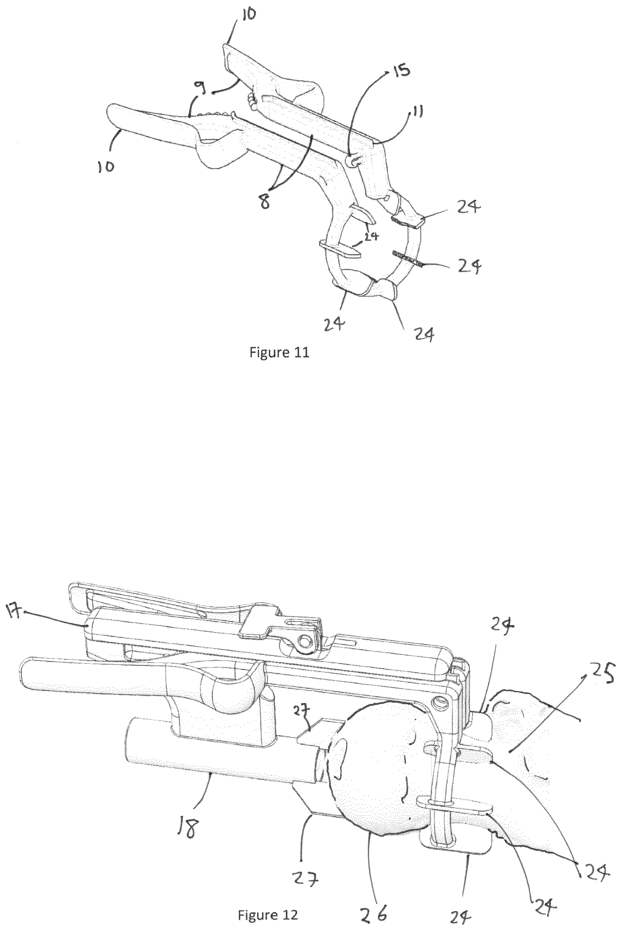

A patient specific version of the head guide is shown in Figures 10 - 13 which differs from the generic version in that it includes the addition of protrusions on both head guide clamp and drill guide. On the head guide clamp protrusions [24] extend from the circular ring [4] to fit a specific patient's femoral neck bone surfaces, so that when positioned around the femoral neck [25], the head guide clamp will only close successfully when in a predefined orientation relative to the patient's femur. On the drill guide, protrusions [27] extend from the proximal end of the cylindrical boss [18], also to fit the femoral head [26] in a predefined orientation. In Figure 12, it is apparent how the sliding adjustment in the guide and the ability to lock the drill guide [2] relative to the head guide clamp [1] enables both sets of protrusions [24 & 27] to be in contact with the femoral head [26] and neck [25] simultaneously, forming a stable platform for drilling into the femoral head neck.

An alternative embodiment of the removable drill guide is shown in Figure 14. In this embodiment the removable drill guide [28] incorporates a larger diameter cylindrical boss [29] with a large aperture [30] and removable inner collar [not shown]. The inner collar is for guiding the small diameter drill (for example a 5 mm drill) into the femur bone but when the inner sleeve is removed, a larger counterbore cutter [31] (for example a diameter 35 mm cutter) is introduced and guided by the large aperture [30] in the cylindrical boss. Furthermore, the distal end of the cylindrical boss [32] provides an end stop for a flange [33] on the large counter bore cutter [31]. Therefore, provided the patient specific protrusions [27] on the removable drill guide [28] are in contact with the femoral head surface [26], the depth of counterbore is guided to a predetermined depth relative to the patient's femur bone. The counterbore surface coincides with the internal end face of the resurfacing head implant [not shown] so that the proximal distal position of the resurfacing head implant is precisely controlled by the guide. In this case both the proximal distal axis and proximal distal position are controlled by the guide.

## Claims

1. A femoral head guide clamp (1) comprising:
(a) jaws (6) comprising a ring (4) forming an aperture, the aperture shaped such that (i) the jaws (6) have a closed position in which the aperture fits around a femoral neck of a patient but is too small to accept a femoral head of a patient, and (ii) the jaws (6) are movable from the closed position to an open position in which the aperture is expanded such that it is large enough to allow the femoral head to pass through; wherein the ring (4) comprises a discontinuity (5), the ring (4) being openable at the discontinuity (5) when the jaws (6) are moved towards the open position in order to allow the femoral head to pass through the ring (4), and
(b) two opposing arms (7) that extend from the jaws (6) in a direction perpendicular to the plane of the ring (4) and which have a proximal section (8) in which the arms (7) are substantially parallel in a side-by-side arrangement, and a distal section (9) in which the arms (7) extend away from each other such that moving the two distal sections (9) towards each other moves the jaws (6) towards the open position.

2. A femoral head guide clamp (1) as claimed in claim 1, wherein the jaws (6) include a lockable member (15,16) for securing the jaws (6) in the closed position.

3. A femoral head guide clamp (1) as claimed in claim 1, wherein the jaws (6) extend away from the ring (4) in a direction which is substantially coplanar with the ring (4), and then connect to the arms (7) which extend distally in a direction which, in use, is towards a user of the femoral head guide clamp.

4. A femoral head guide clamp as claimed in any one of the preceding claims, wherein the distal section (9) of at least one of the two opposing arms (7) comprises protrusions which are shaped to be received by corresponding depressions on the opposing arm when the two distal sections (9) are moved towards each other to move the jaws (6) towards the open position.

5. A femoral head guide clamp as claimed in any one of the preceding claims additionally comprising a guide for a drill or cutter.

6. A femoral head guide clamp as claimed in claim 5, wherein the guide for a drill or cutter is removable.

7. A femoral head guide clamp as claimed in claim 6, wherein the removable guide is slidably attachable to the two opposing arms of the femoral head guide clamp.

8. A femoral head guide clamp as claimed in claim 7, wherein the two opposing arms each comprise an elongate rail extending along each arm and which are shaped to mate with two corresponding elongate recesses on the removable guide.

9. A femoral head guide clamp as claimed in claim 8, wherein the removable guide comprises a lockable member for securing the removable guide to the femoral head guide clamp, the lockable member being able to press the elongate rails against a wall of each of the elongate recesses in order to secure the removable guide to the femoral head guide clamp.

10. A femoral head guide clamp as claimed in any one of the preceding claims, wherein one or both of the femoral head guide clamp and the removable guide is a patient specific instrument.

11. A femoral head guide clamp as claimed in claim 10, wherein the jaws are provided with one or more neck protrusions which are shaped such that the jaws are only moveable to the closed position when around a femoral neck of a specific patient and when the femoral head guide clamp is in a predefined orientation relative to that patient's femur.

12. A femoral head guide clamp as claimed in claim 11, wherein the neck protrusions are provided on the ring.

13. A femoral head guide clamp as claimed in claim 12, wherein the neck protrusions extend in one or more of the following directions: above a plane of the ring, below a plane of the ring, towards a centre of the ring, and away from a centre of the ring.

14. A femoral head guide clamp as claimed in any one of claims 10-13 when dependent on any one of claims 5-9, the guide for a drill or cutter comprising one or more head protrusions which, in use, extend towards a femoral head of a specific patient and which are shaped to conform to the surface of the femoral head when the jaws are closed around a femoral neck of the patient.

## Patentansprüche

1. Femurkopfführungsklemme (1), umfassend:
(a) Backen (6), die einen Ring (4) umfassen, der einen Durchlass ausbildet, wobei der Durchlass derart geformt ist, dass (i) die Backen (6) eine geschlossene Position aufweisen, in der der Durchlass um einen Femurhals eines Patienten passt, aber zu klein ist, um einen Femurkopf eines Patienten aufzunehmen, und (ii) die Backen (6) aus der geschlossenen Position in eine offene Position bewegbar sind, in der der Durchlass derart erweitert ist, dass er groß genug ist, um den Femurkopf hindurch verlaufen zu lassen; wobei der Ring (4) eine Diskontinuität (5) umfasst, wobei der Ring (4) an der Diskontinuität (5) öffenbar ist, wenn die Backen (6) zu der offenen Position hin bewegt werden, um den Femurkopf durch den Ring (4) verlaufen zu lassen, und
(b) zwei gegenüberliegende Arme (7), die sich von den Backen (6) in einer Richtung senkrecht zu der Ebene des Rings (4) erstrecken und die einen proximalen Abschnitt (8), in dem die Arme (7) in einer nebeneinanderliegenden Anordnung im Wesentlichen parallel sind, und einen distalen Abschnitt (9), in dem sich die Arme (7) derart voneinander weg erstrecken, dass ein Bewegen der zwei distalen Abschnitte (9) zueinander hin die Backen (6) zu der offenen Position hin bewegt, aufweisen.

2. Femurkopfführungsklemme (1) nach Anspruch 1, wobei die Backen (6) ein verriegelbares Element (15, 16) zum Sichern der Backen (6) in der geschlossenen Position einschließen.

3. Femurkopfführungsklemme (1) nach Anspruch 1, wobei sich die Backen (6) von dem Ring (4) weg in einer Richtung erstrecken, die im Wesentlichen komplanar mit dem Ring (4) ist, und sich dann mit den Armen (7) verbinden, die sich distal in einer Richtung erstrecken, die sich in Verwendung zu einem Benutzer der Femurkopfführungsklemme hin erstreckt.

4. Femurkopfführungsklemme nach einem der vorhergehenden Ansprüche, wobei der distale Abschnitt (9) mindestens eines der zwei gegenüberliegenden Arme (7) Vorsprünge umfasst, die so geformt sind, dass sie durch entsprechende Vertiefungen an dem gegenüberliegenden Arm aufgenommen werden, wenn die zwei distalen Abschnitte (9) zueinander hin bewegt werden, um die Backen (6) zu der offenen Position hin zu bewegen.

5. Femurkopfführungsklemme nach einem der vorhergehenden Ansprüche, zusätzlich umfassend eine Führung für einen Bohrer oder Fräser.

6. Femurkopfführungsklemme nach Anspruch 5, wobei die Führung für einen Bohrer oder Fräser entfernbar ist.

7. Femurkopfführungsklemme nach Anspruch 6, wobei die entfernbare Führung verschiebbar an den zwei gegenüberliegenden Armen der Femurkopfführungsklemme anbringbar ist.

8. Femurkopfführungsklemme nach Anspruch 7, wobei die zwei gegenüberliegenden Arme jeweils eine längliche Schiene umfassen, die sich entlang jedes Arms erstrecken und die so geformt sind, dass sie mit zwei entsprechenden länglichen Aussparungen an der entfernbaren Führung zusammenpassen.

9. Femurkopfführungsklemme nach Anspruch 8, wobei die entfernbare Führung ein verriegelbares Element zum Sichern der entfernbaren Führung an der Femurkopfführungsklemme umfasst, wobei das verriegelbare Element in der Lage ist, die länglichen Schienen gegen eine Wand jeder der länglichen Aussparungen zu drücken, um die entfernbare Führung an der Femurkopfführungsklemme zu sichern.

10. Femurkopfführungsklemme nach einem der vorhergehenden Ansprüche, wobei eine oder beide der Femurkopfführungsklemme und der entfernbaren Führung ein patientenspezifisches Instrument sind.

11. Femurkopfführungsklemme nach Anspruch 10, wobei die Backen mit einem oder mehreren Halsvorsprüngen versehen sind, die derart geformt sind, dass die Backen nur dann in die geschlossene Position bewegbar sind, wenn sie sich um einen Femurhals eines spezifischen Patienten befinden und wenn sich die Femurkopfführungsklemme in einer vordefinierten Ausrichtung relativ zu dem Femur dieses Patienten befindet.

12. Femurkopfführungsklemme nach Anspruch 11, wobei die Halsvorsprünge an dem Ring bereitgestellt sind.

13. Femurkopfführungsklemme nach Anspruch 12, wobei sich die Halsvorsprünge in eine oder mehrere der folgenden Richtungen erstrecken: oberhalb einer Ebene des Rings, unterhalb einer Ebene des Rings, zu einer Mitte des Rings hin und von einer Mitte des Rings weg.

14. Femurkopfführungsklemme nach einem der Ansprüche 10-13, wenn abhängig von einem der Ansprüche 5-9, wobei die Führung für einen Bohrer oder Fräser einen oder mehrere Kopfvorsprünge umfasst, die sich in Verwendung zu einem Femurkopf eines spezifischen Patienten hin erstrecken und die so geformt sind, dass sie sich an die Oberfläche des Femurkopfes anpassen, wenn die Backen um einen Femurhals des Patienten geschlossen sind.

## Revendications

1. Pince de guidage de tête fémorale (1) comprenant :
(a) des mâchoires (6) comprenant une bague (4) formant une ouverture, l'ouverture étant formée de sorte que (i) les mâchoires (6) aient une position fermée dans laquelle l'ouverture s'adapte autour d'un col fémoral d'un patient mais est trop petite pour recevoir une tête fémorale d'un patient, et (ii) les mâchoires (6) sont mobiles de la position fermée à une position ouverte dans laquelle l'ouverture est dilatée de sorte qu'elle soit suffisamment grande pour permettre le passage de la tête fémorale ; ladite bague (4) comprenant une discontinuité (5), ladite bague (4) pouvant être ouverte au niveau de la discontinuité (5) lorsque les mâchoires (6) sont déplacées vers la position ouverte afin de permettre le passage de la tête fémorale à travers la bague (4), et
(b) deux bras opposés (7) qui s'étendent à partir des mâchoires (6) dans une direction perpendiculaire au plan de la bague (4) et qui possèdent une section proximale (8) dans laquelle les bras (7) sont sensiblement parallèles dans un agencement côte à côte, et une section distale (9) dans laquelle les bras (7) s'éloignent l'un de l'autre de sorte que le déplacement des deux sections distales (9) l'une vers l'autre déplace les mâchoires (6) vers la position ouverte.

2. Pince de guidage de tête fémorale (1) selon la revendication 1, lesdites mâchoires (6) comprenant un élément verrouillable (15, 16) destiné à fixer les mâchoires (6) dans la position fermée.

3. Pince de guidage de tête fémorale (1) selon la revendication 1, lesdites mâchoires (6) s'éloignant de la bague (4) dans une direction qui est sensiblement coplanaire avec la bague (4), puis se raccordant aux bras (7) qui s'étendent distalement dans une direction qui, lors de l'utilisation, est vers un utilisateur de la pince de guidage de tête fémorale.

4. Pince de guidage de tête fémorale selon l'une quelconque des revendications précédentes, ladite section distale (9) d'au moins l'un des deux bras opposés (7) comprenant des saillies qui sont formées de manière à être reçues par des dépressions correspondantes sur le bras opposé lorsque les deux sections distales (9) sont déplacées l'une vers l'autre afin de déplacer les mâchoires (6) vers la position ouverte.

5. Pince de guidage de tête fémorale selon l'une quelconque des revendications précédentes, comprenant en outre un guide pour un foret ou une fraise.

6. Pince de guidage de tête fémorale selon la revendication 5, ledit guide destiné à un foret ou une fraise étant amovible.

7. Pince de guidage de tête fémorale selon la revendication 6, ledit guide amovible pouvant être fixé de manière coulissante aux deux bras opposés de la pince de guidage de tête fémorale.

8. Pince de guidage de tête fémorale selon la revendication 7, lesdits deux bras opposés comprenant chacun un rail allongé s'étendant le long de chaque bras et qui sont formés de manière à s'accoupler à deux évidements allongés correspondants sur le guide amovible.

9. Pince de guidage de tête fémorale selon la revendication 8, ledit guide amovible comprenant un élément verrouillable destiné à fixer le guide amovible à la pince de guidage de tête fémorale, ledit élément verrouillable étant capable de presser les rails allongés contre une paroi de chacun des évidements allongés afin de fixer le guide amovible à la pince de guidage de tête fémorale.

10. Pince de guidage de tête fémorale selon l'une quelconque des revendications précédentes, l'un ou les deux éléments parmi la pince de guidage de tête fémorale et le guide amovible étant un instrument spécifique au patient.

11. Pince de guidage de tête fémorale selon la revendication 10, lesdites mâchoires étant pourvues d'une ou de plusieurs saillies de col qui sont formées de sorte que les mâchoires ne puissent être déplacées vers la position fermée qu'autour d'un col fémoral d'un patient spécifique et lorsque la pince de guidage de tête fémorale se trouve dans une orientation prédéfinie par rapport au fémur de ce patient.

12. Pince de guidage de tête fémorale selon la revendication 11, lesdites saillies de col étant ménagées sur la bague.

13. Pince de guidage de tête fémorale selon la revendication 12, lesdites saillies de col s'étendant dans une ou plusieurs des directions suivantes : au-dessus d'un plan de la bague, en dessous d'un plan de la bague, vers le centre de la bague, et à l'écart du centre de la bague.

14. Pince de guidage de tête fémorale selon l'une quelconque des revendications 10 à 13, lorsqu'elle dépend de l'une quelconque des revendications 5 à 9, ledit guide destiné à un foret ou une fraise comprenant une ou plusieurs saillies de tête qui, lors de l'utilisation, s'étendent vers une tête fémorale d'un patient spécifique et qui sont formées de manière à se conformer à la surface de la tête fémorale lorsque les mâchoires sont fermées autour d'un col fémoral du patient.
